# EUROPEAN PATENT SPECIFICATION

(11) **EP 3 453 378 B1**
(45) Date of publication and mention of the grant of the patent: **25.03.2020**
(21) Application number: 18193157.7
(22) Date of filing: 07.09.2018
(51) Int. Cl.: A61K 8/39, A61K 8/49, A61K 8/73, A61Q 19/06, A61K 8/19, A61P 3/04

(54) **A COSMETIC GEL COMPOSITION**
EINE KOSMETISCHE GEL-ZUSAMMENSETZUNG
COMPOSITION COSMETIQUE DE GEL

(30) Priority: 07.09.2017 IT 201700100395
(43) Date of publication of application: 13.03.2019
(73) Proprietor: Società Italo Britannica L. Manetti - H. Roberts & C.p.A., 50123 Firenze (IT)
(72) Inventor: OLIVA, Marco, 50129 Firenze (IT); MANNUCCI, Emanuela, 50129 Firenze (IT); BORRUTO, Michela, 51100 Pistoia (IT); CENNI, Anna, 50142 Firenze (IT)
(74) Representative: Brazzini, Silvia

(56) References cited:
- EP-A1- 2 305 206
- JP-A- 2005 336 068
- US-A1- 2004 166 079
- US-A1- 2008 200 534
- DATABASE GNPD [Online] MINTEL; June 2017 (2017-06), "Pore Purifyinig Cleansing Jelly", XP002778890, Database accession no. 4861319

## Description

### Field of the Invention

The present invention relates in general to the cosmetic field, and more precisely it relates to a cosmetic gel composition having an effective slimming action, to the process for its preparation and to a cosmetic method comprising its application on the skin.

### State of the Art

Among the most wanted cosmetic products by consumers, there are definitely the formulations of treatment that achieve slimming results through the simple application at home of the product on the skin, without the need of medical treatments or of special formulations that can only be administered in beauty centres.

Examples of known compositions of this type are the compositions based on inorganic salts, which are known to have the ability to drain away the accumulated liquids in the skin layer with a consequent deflating and slimming effect. In this sense, these compositions are often presented as anti-cellulite products, since the onset of cellulitis is just linked to the accumulation of water in the extracellular interstices of the cutaneous layer, therefore draining away such liquids has a positive effect on the possible incipient cellulitis.

However, in order to obtain a satisfactory draining effect of the liquids, the concentration of inorganic salts in such compositions must be rather high, but this creates a series of problems in the formulation of the product, linked in particular to the stability of the formulation. To date these problems have been addressed by proposing practically liquid products, or pasty emulsions, with emulsifiers and a relevant fat component. However, both these types of compositions have defects affecting the consumer's satisfaction: the product in emulsion form has a low spreadability and gives a feeling of heaviness on the skin, while the liquid product is certainly more pleasant but it does not stay on the skin for enough time to carry out the desired action.

For the above said reasons, today in the field the need is felt for slimming products with a high concentration of inorganic salts but having a formulation different from those known up to now, which in particular combines freshness and lightness on the skin, better spreadability and good skin permeability to achieve the desired slimming effect.

### Summary of the Invention

Now the Applicant has found a novel cosmetic composition in gel form based on inorganic salts and having an effective slimming action, which solves the issues described above for the known products. As a matter of fact, on one hand the present composition provides freshness and lightness on the skin, but also assuring on the other hand a fair residence time on the skin.

At the same time, the cosmetic composition of the invention is furthermore perfectly stable and able to incorporate a further cosmetically active principle with slimming action besides the inorganic salts, the caffeine, without this compromising the stability and the characteristics described above of effectiveness and pleasantness of the composition.

Subject of the invention are therefore gel cosmetic compositions with slimming action, whose essential characteristics are defined in the first of the herewith attached claims. Further important characteristics of the compositions according to the invention are defined in the claims dependent from the first one.

A further subject of the invention is a process for the preparation of the above said compositions, whose essential characteristics are defined by the related independent claim attached herewith.

Still a further subject of the invention is a cosmetic slimming method comprising the treatment of the cutaneous layer with the above said composition.

Further important features of the compositions, of the process for their preparation and of the cosmetic method according to the invention are disclosed in the following detailed description.

### Detailed Description of the Invention

According to the present invention a gel cosmetic composition is herein disclosed having a viscosity ranging between 50,000 and 200,000 mPa*s comprising:
inorganic salt in amount ranging between 8 and 15% by weight with respect to the total weight of the composition;
caffeine in amount ranging between 0.5 and 3% by weight;
ethanol in amount ranging between 5 and 20% by weight; and
an association of xanthan gum with a cellulose derivative in amount ranging between 1 and 6% by weight,
wherein the inorganic salt is sea salt or pure sodium chloride.

The above described cosmetic composition of this invention is free from agents having an emulsifying action and from fat components.

Within the present invention, by the term "viscosity" is meant the viscosity expressed in cPs, measured at the temperature of 20°C and under atmospheric pressure with a rotary viscometer.

Preferably, the present cosmetic composition comprises sea salt as the inorganic salt having a draining action of the liquids in the cutaneous layer.

Within the present invention by the term "sea salt" the sodium chloride is meant obtained from evaporation of the sea water, comprising in variable percentages other inorganic salts too, in particular magnesium and potassium salts.

By "fat components" that are excluded from the cosmetic composition of this invention are meant the compounds or mixtures of compounds so-called "fat", i.e. having in their structure long aliphatic chains of carbon atoms, typically chains having C>14, that can be easily identified by any persons with ordinary skills in the art. By way of a non-limiting example, fatty components may include, for example, synthetic or vegetable oils, butters, waxes, lanolin, petrolatum, fatty alcohols, and mixtures thereof.

In a preferred embodiment of the present invention the cellulose derivative is selected from the group consisting of hydroxyethyl cellulose, methyl cellulose, ethyl cellulose, methyl ethyl cellulose, methyl hydroxyethyl cellulose, hydroxybutyl methyl cellulose, hydroxypropyl methyl cellulose, hydroxyethyl ethyl cellulose and hydroxypropyl cellulose. According to a particularly preferred embodiment of the invention, hydroxyethyl cellulose is used as cellulose derivative in association with xanthan gum.

According to the invention, the association of xanthan gum and cellulose derivative that contributes to the obtainment of the composition's desired viscosity and to the formulation of the high salt concentration in a stable composition, can for example have a weight ratio between xanthan gum and cellulose derivative comprised between 0.6 and 2.0. The optimal viscosity of the present composition, equal to approximately 100,000 mPa*s, is obtained with a weight ratio between xanthan gum and cellulose derivative of approximately 1.6.

The composition of the invention as defined above provides a stable gel which is transparent or at least translucent and has a viscosity in the range indicated above.

In spite of the absence of emulsifying agents and fat components, the present composition surprisingly succeeds in particular in solubilizing and maintaining in a stable form in the formulation of ethanol and xanthan gum / cellulose derivative also the caffeine, a known lipolytic active ingredient, also known for the difficulty in being formulated in cosmetic compositions. In fact, caffeine tends to crystallize and form needles that can be perceived in the final product: this is not acceptable from both the point of view of the pleasantness of a cosmetic formulation and the point of view of its effectiveness.

The present compositions, besides the essential components described above, may further comprise fragrances, preservatives, conditioning agents, wetting/moisturising agents, colouring agents and/or any other known additive commonly used in the field. In particular, the present compositions may comprise as optional ingredients one or more softening agents, which are generally classified as wetting/moisturising agents, in amounts that may vary between 0 and approximately 20% by weight with respect to the total weight of the composition. Examples of wetting/moisturising agents are selected from the group consisting of glycerine, glycols, and mixtures thereof.

Besides the above mentioned essential ingredients, and to the optional softening components, the compositions of the invention can optionally comprise also silicones, functional ingredients and claim ingredients. By functional ingredients and claim ingredients according to the present invention are meant respectively active ingredients having a cosmetic action, incorporated in the composition at functional doses and substances whose presence in the composition is claimed, generally in limited amount, by explicitly indicating their action or implicitly referring to their beneficial properties. Examples of functional and/or claim ingredients according to the invention are for example plant extracts or active principles with slimming action and/or with activity on the blood microcirculation. These optional agents described above, as a whole, are typically present in the compositions of the invention in amount ranging from 0 to approximately 5% by weight with respect to the total weight of the composition.

Within the present invention, wherein not specified, the weight percentages indicating the amount of the different ingredients are to be meant as weight percentages of the ingredient in question with respect to the total weight of the composition.

The formulation of the present compositions, even if free from emulsifying agents and fat components, succeeds in maintaining solubilised and stable the active components, in particular the caffeine, and the high concentration of inorganic salt. It also has an optimal viscosity for a good spreadability and a more appropriate residence on the skin to be treated with respect for example to the products of the state of the art in liquid formulations.

Even with respect to the products of the known art in cream or in emulsion form, the present composition has the advantage of a better spreadability and pleasantness on the skin, due to the marked freshness and lightness of the product in gel form. In this sense in particular, the presence of ethanol, besides contributing to the solubilization of the active ingredients, makes the formulation particularly fresh and pleasant on the skin, also improving the skin permeability to the active principles.

For these reasons, it can be said that the composition of the invention, in addition to being really effective in its activity of draining liquids and of slimming as shown by the experimental data described below, is optimal from every point of view and allows overcoming all the drawbacks highlighted above in describing the state of the art.

As far as the preparation of the compositions of the invention is concerned, the process implemented by the Applicant comprises the following steps:
i) formation of an aqueous solution of caffeine;
ii) addition to the aqueous solution of step i) of the association between xanthan gum and cellulose derivative up to a gel formation;
iii) addition of the inorganic salt to the gel formed in the step ii);
iv) addition of ethanol and possibly of other optional components, as such or already dissolved in water or in ethanol.

Any person with ordinary skills in the art will be able to select, easily and without any undue burden, the conditions of agitation, temperature, pre-solubilisation in water or alcohol of the optional components for addition in step iv) in order to assist the solubilisation of all components and the formation of a stable and uniform gel.

The mixing operations of the different components are carried out in special containers suitable for the production of cosmetic products, previously washed and sanitized, equipped with common means for stirring the mixtures.

The present cosmetic compositions, so prepared, can be packed in jar containers or distributed by a pump dispenser, typically used for the cosmetic gels.

The following examples are provided as a non-limiting illustration of the present invention.

### EXAMPLE 1

In the following table the components of an exemplary composition of the above described invention are summarised with their respective amounts:

| **Component** | **Amount% (w/w)** |
|---|---|
| Demineralised water | Qb a 100 |
| Sea salt | 12 |
| Denatured absolute ethanol, category A | 8 |
| Glycerol | 5 |
| Dimethicone 92%, dimethicone/vinyl dimethicone crosspolymer 8% | 1.5 |
| Xanthan gum | 1.0 |
| Hydroxyethyl cellulose | 1.5 |
| Caffeine | 1.0 |
| Fragrance | 1 |
| Sodium benzoate | 0.5 |
| Menthol | 0.3 |
| Imidazolinyl urea | 0.3 |
| Colouring agent powder blue | 0.00125 |

In a sanitised recipient of appropriate size, equipped with mechanical agitation means, water, caffeine, sodium benzoate and the carrier ethoxydiglycol have been introduced in the proportions indicated above in table. Then the mixture was mixed and heated up to about 80°C. Then the association of xanthan gum and hydroxyethyl cellulose was added keeping the mixture under agitation until a uniform gel is formed. Still under agitation the salt was added then glycerol and siliconic product, letting then the mixture to cool down to 35°C.

The additional components have been added as such or as a premixture with water or with ethanol, finally the colouring agent was added and the mixing has continued until a perfectly homogeneous product was obtained.

The gel product obtained as described above was in fact perfectly smooth and homogeneous, with a blue translucent appearance. It was characterised as having a viscosity at the end of the processing at temperature lower than 30°C of about 80,000-120,000 cPs and a density of 1.05-1.06 g/cm³.

In particular, the viscosity was measured by a rotary viscometer Brookfield RVT type. A sample product in gel form obtained as described above was placed in a 250 ml Becker and brought to a temperature of 20°C ± 1°C, then a TE type rotor was immersed in the sample setting its rotation speed at 5 rpm. After a few seconds, the reading on the instrument's display was stable and has provided the relative viscosity value of the sample.

The slimming properties of this product have been evaluated with an instrumental clinical trial (double-blind vs. placebo) performed by an independent dermatological Institute. The test was carried out on 102 women in total by measuring, with appropriate instruments, several objective parameters, such as circumference, thickness of the adipose panniculus of the treated area and volume of the interstitial fluid, on different areas of the body, in particular waist circumference, hips and thighs.

The slimming efficacy was evaluated by comparing the results obtained after 28 days of overnight treatment with the initial values measured, carrying out intermediate controls after 7 and 15 days. By overnight treatment is meant that the product has been distributed on the area to be treated before the night rest and it has been left to act for the whole night.

The study showed that the product acts on the localized fat and on the skin fluids in excess with first results of slimming already after only 7 nights of treatment. The study also highlighted that, by continuing the treatment beyond the first 7 nights, the slimming efficiency increases up to double after 1 month of treatment.

## Claims

1. A gel cosmetic composition having a viscosity ranging between 50,000 and 200,000 mPa*s measured at 20°C and under atmospheric pressure with a rotary viscometer, comprising:
- inorganic salt in amount ranging between 8 and 15% by weight with respect to the total weight of the composition;
- caffeine in amount ranging between 0.5 and 3% by weight;
- ethanol in amount ranging between 5 and 20% by weight; and
- an association of xanthan gum with a cellulose derivative in amount ranging between 1 and 6% by weight,
wherein the inorganic salt is sea salt or pure sodium chloride.

2. The cosmetic composition according to claim 1, wherein in said association of xanthan gum and cellulose derivative the ratio between xanthan gum and cellulose derivative is comprised between 0.6 and 2.0.

3. The cosmetic composition according to claim 1, wherein in said association of xanthan gum and cellulose derivative the ratio between xanthan gum and cellulose derivative is of 1.6.

4. The cosmetic composition according to any one of the preceding claims, wherein the cellulose derivative is selected from the group consisting of hydroxyethyl cellulose, methyl cellulose, ethyl cellulose, methyl ethyl cellulose, methyl hydroxyethyl cellulose, hydroxybutyl methyl cellulose, hydroxypropyl methyl cellulose, hydroxyethyl ethyl cellulose and hydroxypropyl cellulose.

5. The cosmetic composition according to claim 4, wherein the cellulose derivative is hydroxyethyl cellulose.

6. The cosmetic composition according to any one of the preceding claims, wherein the inorganic salt is in amount of 12% by weight.

7. The cosmetic composition according to any one of the preceding claims, wherein caffeine is in amount of 1.0% by weight.

8. The cosmetic composition according to any one of the preceding claims, wherein ethanol is in amount of 8% by weight.

9. The cosmetic composition according to any one of the preceding claims, further comprising one or more further ingredients selected from among fragrances, colouring agents, wetting/moisturising agents, preservatives, functional and/or claim ingredients, and mixtures thereof.

10. The cosmetic composition according to any one of the preceding claims, comprising:
| **Component** | **Amount% (w/w)** |
|---|---|
| Demineralised water | As much as suffices to 100 |
| Sea salt | 12 |
| Denatured absolute ethanol, category A | 8 |
| Glycerol | 5 |
| Dimethicone 92%, dimethicone/vinyl dimethicone crosspolymer 8% | 1.5 |
| Xanthan gum | 1.0 |
| Hydroxyethyl cellulose | 1.5 |
| Caffeine | 1.0 |
| Fragrance | 1 |
| Sodium benzoate | 0.5 |
| Menthol | 0.3 |
| Imidazolinyl urea | 0.3 |
| Colouring agent powder blue | 0.00125 |

11. A process for the preparation of a cosmetic composition as defined in the claims 1-10, comprising the following steps:
i) preparing an aqueous solution of caffeine;
ii) adding to the aqueous solution of step i) said association xanthan gum and cellulose derivative until a gel is formed;
iii) adding an inorganic salt to the gel formed in the step ii);
iv) adding ethanol and possibly other optional components, as such or dissolved in water or in ethanol.

12. A cosmetic slimming method comprising a step of treatment of the skin layer of a subject with the cosmetic composition as defined in the claims 1-10.

## Patentansprüche

1. Kosmetische Gel-Zusammensetzung, die eine Viskosität hat, die im Bereich zwischen 50.000 und 200.000 mPa * s liegt, gemessen bei 20 °C und unter atmosphärischem Druck mit einem Drehviskometer, wobei enthalten sind:
- anorganisches Salz in einer Menge, die im Bereich zwischen 8 und 15 Gewichts-% bezüglich dem totalen Gewicht der Zusammensetzung liegt;
- Koffein in einer Menge, die im Bereich zwischen 0,5 und 3 Gewichts-% liegt;
- Ethanol in einer Menge, die im Bereich zwischen 5 und 20 Gewichts-% liegt; und
- eine Verbindung von Xanthan-Gummi mit einem Zellulosederivat in einer Menge, die im Bereich zwischen 1 und 6 Gewichts-% liegt;
wobei das anorganische Salz Meersalz oder reines Natriumchlorid ist.

2. Kosmetische Zusammensetzung nach Anspruch 1, wobei in der Verbindung von Xanthan-Gummi und Zellulosederivat das Verhältnis zwischen Xanthan-Gummi und Zellulosederivat zwischen 0,6 und 2,0 enthalten ist.

3. Kosmetische Zusammensetzung nach Anspruch 1, wobei in der Verbindung von Xanthan-Gummi und Zellulosederivat das Verhältnis zwischen Xanthan-Gummi und Zellulosederivat 1,6 ist.

4. Kosmetische Zusammensetzung nach einem der vorhergehenden Ansprüche, wobei das Zellulosederivat ausgewählt ist aus der Gruppe, die besteht aus Hydroxyethyl-Zellulose, Methyl-Zellulose, Ethyl-Zellulose, Methyl-Ethyl-Zellulose, Methyl-Hydroxyethyl-Zellulose, Hydroxybutyl-Methyl-Zellulose, Hydroxypropyl-Methyl-Zellulose, Hydroxyethyl-Ethyl-Zellulose und Hydroxypropyl-Zellulose.

5. Kosmetische Zusammensetzung nach Anspruch 4, wobei das Zellulosederivat Hydroxyethyl-Zellulose ist.

6. Kosmetische Zusammensetzung nach einem der vorhergehenden Ansprüche, wobei das anorganische Salz in einer Menge von 12 Gewichts-% ist.

7. Kosmetische Zusammensetzung nach einem der vorhergehenden Ansprüche, wobei Koffein in einer Menge von 1,0 Gewichts-% ist.

8. Kosmetische Zusammensetzung nach einem der vorhergehenden Ansprüche, wobei Ethanol in einer Menge von 8 Gewichts-% ist.

9. Kosmetische Zusammensetzung nach einem der vorhergehenden Ansprüche, wobei ferner ein oder mehrere weitere(r) Bestanteil(e) unter Duftstoffen, Färbungsmitteln, Benetzungs-/Befeuchtungsmittel, Konservierungsmittel, funktionale und/oder Anforderungsbestanteile und Mischungen davon ausgewählt ausgewählt ist/sind.

10. Kosmetische Zusammensetzung nach einem der vorhergehenden Ansprüche, wobei enthalten ist:
| **Komponente** | **Menge % (w/w)** |
|---|---|
| Demineralisiertes Wasser | soviel wie bis 100 genügt |
| Meersalz | 12 |
| Denaturiertes absolutes Ethanol, Kategorie A | 8 |
| Glycerol | 5 |
| Dimethikon 92 %, Dimethikon/Vinyl-Dimethikon Kreuzpolymer 8 % | 1,5 |
| Xanthan-Gummi | 1,0 |
| Hydroxyethyl-Zellulose | 1,5 |
| Koffein | 1,0 |
| Duftstoff | 1 |
| Natriumbenzoat | 0,5 |
| Menthol | 0,3 |
| Imidazolinyl-Harnstoff | 0,3 |
| Färbungsmittelpulver bkau | 0,00125 |

11. Verfahren für die Herstellung einer kosmetischen Zusammensetzung, wie sie in den Ansprüchen 1 bis 10 definiert ist, wobei die folgenden Schritte enthalten sind:
i) Vorbereiten einer wässrigen Lösung von Koffein;
ii) Hinzufügen der Verbindung Xanthan-Gummi und Zellulosederivate zu der wässrigen Lösung von Schritt i), bis ein Gel gebildet wurde;
iii) Hinzufügen eines anorganischen Salzes zu dem Gel, das im Schritt ii) gebildet wurde;
iv) Hinzufügen von Ethanol und möglicherweise anderen optionalen Komponenten als solche oder gelöst in Wasser oder Ethanol.

12. Kosmetisches Abnehmverfahren, wobei ein Schritt der Behandlung der Hautschicht eines Subjektes mit der kosmetischen Zusammensetzung enthalten ist, die in den Ansprüchen 1 bis 10 definiert ist.

## Revendications

1. Une composition cosmétique en gel ayant une viscosité comprise entre 50.000 et 200.000 mPa *s mesurée à 20°C et sous pression atmosphérique avec un viscosimètre rotatif, comprenant :
- du sel inorganique en quantité comprise entre 8 et 15% en poids par rapport au poids total de la composition ;
- de la caféine en quantité comprise entre 0,5 et 3% en poids ;
- de l'éthanol en quantité comprise entre 5 et 20% en poids ; et
- une association de gomme de xanthane avec un dérivé de cellulose en quantité comprise entre 1 et 6% en poids,
dans laquelle le sel inorganique est du sel marin ou du chlorure de sodium pur.

2. La composition cosmétique selon la revendication 1, dans laquelle, dans ladite association de gomme de xanthane et de dérivé de cellulose, le rapport entre la gomme de xanthane et le dérivé de cellulose est compris entre 0,6 et 2,0.

3. La composition cosmétique selon la revendication 1, dans laquelle, dans ladite association de gomme de xanthane et de dérivé de cellulose, le rapport entre la gomme de xanthane et le dérivé de cellulose est d'environ 1,6.

4. La composition cosmétique selon l'une quelconque des revendications précédentes, dans laquelle le dérivé de cellulose est choisi dans le groupe consistant en hydroxyéthylcellulose, méthylcellulose, éthylcellulose, méthyléthylcellulose, méthylhydroxyéthylcellulose, hydroxybutylméthylcellulose, hydroxypropylméthylcellulose, hydroxyéthyléthylcellulose et hydroxypropylcellulose.

5. La composition cosmétique selon la revendication 4, dans laquelle le dérivé de cellulose est l'hydroxyéthylcellulose.

6. La composition cosmétique selon l'une quelconque des revendications précédentes, dans laquelle le sel inorganique est en quantité d'environ 12% en poids.

7. La composition cosmétique selon l'une quelconque des revendications précédentes, dans laquelle la caféine est en quantité d'environ 1,0% en poids.

8. La composition cosmétique selon l'une quelconque des revendications précédentes, dans laquelle l'éthanol est en quantité d'environ 8% en poids.

9. La composition cosmétique selon l'une quelconque des revendications précédentes, comprenant en outre un ou plusieurs autres ingrédients choisis parmi des parfums, des agents colorants, des agents mouillants / hydratants, des conservateurs, des ingrédients fonctionnels et/ou d'allégation, et leurs mélanges.

10. La composition cosmétique selon l'une quelconque des revendications précédentes, comprenant :
| **Composant** | **Quantité % (poids/poids)** |
|---|---|
| Eau déminéralisée | Autant qu'il suffit pour 100 |
| Sel marin | 12 |
| Éthanol absolu dénaturé, catégorie A | 8 |
| Glycérol | 5 |
| Diméthicone 92%, diméthicone/vinyl diméthicone polymère réticulé 8% | 1,5 |
| Gomme de xanthane | 1,0 |
| Hydroxyéthylcellulose | 1,5 |
| Caféine | 1,0 |
| Parfum | 1 |
| Benzoate de sodium | 0,5 |
| Menthol | 0,3 |
| Imidazolinyl urée | 0,3 |
| Agent colorant en poudre bleu | 0,00125 |

11. Un procédé pour la préparation d'une composition cosmétique telle que définie dans les revendications 1 à 10, comprenant les étapes suivantes :
i) préparer une solution aqueuse de caféine ;
ii) ajouter à la solution aqueuse de l'étape i) ladite d'association de gomme xanthane et de dérivé cellulosique jusqu'à formation d'un gel ;
iii) ajouter un sel inorganique au gel formé à l'étape ii) ;
iv) ajouter de l'éthanol et éventuellement d'autres composants optionnels, tels quels ou dissous dans l'eau ou dans l'éthanol.

12. Un procédé d'amincissement cosmétique comprenant une étape de traitement de la couche cutanée d'un sujet avec la composition cosmétique telle que définie dans les revendications 1-10.
